# EUROPEAN PATENT SPECIFICATION

(11) **EP 1 210 402 B1**
(45) Date of publication and mention of the grant of the patent: **10.05.2006**
(21) Application number: 00960513.0
(22) Date of filing: 23.08.2000
(51) Int. Cl.: C11D 1/94, C11D 1/90, C11D 3/37, A61K 8/04, A61K 8/46, A61K 8/73, A61K 8/44

(54) **SUSPENDING CLEAR CLEANSING FORMULATION**
KLARE REINIGUNGSFORMULIERUNG
FORMULATION DE NETTOYAGE ET DE SUSPENSION CLAIRE

(30) Priority: 10.09.1999 US 153355 P
(43) Date of publication of application: 05.06.2002
(60) Divisional application: 05027073.5
(73) Proprietor: UNILEVER PLC, London, Greater London EC4P 4BQ (GB); UNILEVER N.V., 3013 AL Rotterdam (NL)
(72) Inventor: MARGOSIAK, Marion, L., Unilever Home &, Trumbull, CT 06611 (US); RAHN, Michael, Alan, Unilever Home &, Trumbull, CT 06611 (US); PAREDES, Rosa, Mercedes, Unilever Home &, Trumbull, CT 06611 (US)
(74) Representative: Mulder, Cornelis Willem Reinier
(86) International application number: PCT/EP2000/008265
(87) International publication number: WO 2001/019946

(56) References cited:
- WO-A1-98/13022
- WO-A1-99/36054
- GB-A- 2 283 754
- US-A- 5 656 257

## Description

The invention relates to a shower gel formulation.

Shower gel formulations which are mild to the skin are well known in the art. Such a formulation may optionally contain skin feel agents, such as cationic polymers. However, when one desires to suspend particulates and/or beads in the formulation, frequently substantial quantities of anionic surfactants have been incorporated, and provide clear solutions. Unfortunately, the addition of such anionic surfactants diminishes the mildness of the shower gel formulation.

U.S. Patent No. 5,656,257 (Fealy et al., issued on August 12, 1997) discloses an anionic shampoo and conditioning composition comprising an oily conditioning agent, a shampooing agent, and an acrylate copolymer, a cationic conditioning agent and water. In this formulation, the acrylate copolymer is used to suspend the anionic shampooing and cationic conditioning agent and prevent it then from inactivating one another. U.S. Patent No. 5,656,257 does not, however, disclose a clear, mild cleansing composition containing a combination of surfactant types, which is capable of suspending beads or other insoluble particulates or gas bubbles.

WO-A-9936054 relates to the use of an amphoteric surface active agent for precipitating a water soluble or water dissoluble cationic polymer from a specified composition, and also the formation of a transparent aqueous composition comprising anionic surface active agent, betaines, a water soluble or water dispersible cationic polymer derived from a polysaccharide and at least one amphoteric surface active agent.

U.S. Patent No. 4,552,685 (Kernstock et al., issued November 12, 1985) discloses examples of useful acrylate polymers and copolymers capable of thickening mild cleansing agents containing amphoteric surfactants and betaines. However, there is no teaching in that patent regarding the compatability of cationic polymer conditioning agents in the formulation, nor the suspending power of the solution for insoluble beads, particulates or gaseous bubbles.

U.S. Patent No. 3,759,861 (Shimokawa, issued September 18, 1973) discloses a clear polymer adhesive complex of an acrylate containing polymer and surfactant used to produce a flocculant. However, there is no disclosure of a shower gel or other cleansing composition containing a cationic conditioning agent or complex which can suspend particulates or gas bubbles.

The present invention comprises a shower gel formulation having a clear appearance and which suspends beads (e.g. agar/TiO2/sunflower oil beads), insoluble particles and gas bubbles while having one or more acrylate copolymers, a betaine or other amphoteric surfactant and a cationic polymer (e.g. guar) present in the formulation.

It is known that anionic acrylates (i.e. Aculyn type acrylates (available from ISP)), being anionic polymers are generally considered to be incompatible with cationic charged ingredients. It is further known that polymeric cationics, as well as some large, bulky quaternary materials, can possibly be incorporated in formulations containing such acrylates. The optimum order of addition in these instances generally requires the acrylate to be neutralised with a base prior to the addition of any cationics.

The applicants have discovered that a clear or transparent product can be produced by either partially neutralising such acrylates prior to cationic addition or after cationic addition. Clarity or transparency is herein defined as having a turbidity less than or equal to 105 NTU (Nephelometric Turbidity Units).

The applicants have further discovered that amphoteric surfactants, such as betaine (which is also cationic in nature and not a true amphoteric), are added to the formulation in the range of 2-15 weight percent, preferably 2-10 weight percent to increase mildness without creating noticeable haziness. Prior art shower gels that suspend beads or particulate matter are primarily composed of anionic surfactant and structurant which in most cases are harsher than the inventive formula.

In a first embodiment, the present invention provides an aqueous, clear cleansing gel that is capable of suspending insoluble material or gas bubbles, comprising:
a. 5 to 30, preferably 8 to 20 weight percent of at least one anionic surfactant;
b. a 2 to 15, preferably 2 to 10 weight percent of at least one amphoteric surfactant;
c. 0.1 to 10, preferably 0.5 to 5 weight percent of at least one at least partially neutralised acrylate copolymer;
d. 0,01 to 5, preferably. 0 ,1 to 2 weight percent of at least one cationic polymer;
e. 0.01 to 5, preferably 0,05 to 3 weight percent of at least one insoluble component selected from the group consisting of beads, particulates, water insoluble liquids and gas bubbles;
f. about 50 to 85 weight percent of water
g. wherein there is a weight percent ratio range of anionic surfactant to amphoteric surfactant of 1.9:1 to 15:1 ;
h. wherein there is a weight percent ratio range of the sum of cationic polymer and amphoteric surfactant to acrylate copolymer of 0.1:1 to 16:1 ; and
wherein the concentration of acrylate copolymer is sufficient to suspend said at least one insoluble component, preferably wherein the viscosity range is between 6, 000 and 20,000 cps, and the pH is in the range of 5.5 to 7. 0.

Amonic surfactants, foam boosters, amphoteric and zwitterionic surfactants, which are useful in the present invention, are described in U.S. Patent No. 5,221,530, issued to Janchitraponvej et al. on June 22, 1993.

Preferably, the anionic surfactant is selected from alkyl sulfates, alkyl ether sulfates, alkyl sulfonates, alkylbenzene sulfonates, alkyl succinates, alkyl sulfosuccinates, alkyl olefin sulfonates, alkyl sarcosinates, octoxynol phosphates nonoxynol phosphates, alkyl taurates, polyoxyethylene sulfates, polyoxyethylene isethionates, alkyl carboxylates and alkyl ether carboxylates, and mixtures thereof.

Preferably, amphoteric surfactant is selected from alkyl betaines, alkyl amino betains, hydroxysultaines, alkyl amphoacetates and alkylampho carboxyglycinates, and mixtures thereof.

Acrylate polymers and copolymers which are useful in the invention include one or more copolymers containing at least one monomer selected from the group consisting of methacrylic acid, acrylic acid, amino acrylic acid, an acrylic acid ester of a C8 -30 alkyl, alkylaryl, aryl, heterocyclic, alkoxyl, alkoxyl alkyl ester of a C8-30 alkyl or alkenyl; either substituted or unsubstituted; a methacrylic acid ester of a C8 -C30 alkyl, alkylaryl, aryl, heterocyclic, alkoxyl, alkoxyl alkyl ester of a C8-30 alkyl, or alkenyl; either substituted or unsubstituted; a C1-4 alkyl acrylate, and a C1-4 methacrylate; either substituted or unsubstituted. Other useful acrylate polymers and copolymers are disclosed in U.S. Patent No. 5,656,257.

Cationic polymers suitable for use in the invention include quaternized guar gums, quaternized phosphate esters, quaternized polysaccharides or their derivatives, quaternized polyamides, quaternized polymeric derivatives of acrylates, methacrylates, acrylamides, methacrylamides or copolymers thereof, and quaternized polymeric derivatives of substituted allyl or vinyl compounds.

The insoluble component is preferably selected from the glass beads, plastic beads, macaroni food products, organic materials, inorganic materials, crystalline solids, oil droplets, vegetable and fruit purees, water insoluble dimethicones, air and gas bubbles, and mixtures thereof.

Preferred acrylate polymers include the following INCI named materials: acrylates/c12-24 pareth-25 acrylate copolymer, obtainable as Synthalen® W2000 from 3V Inc. (Wehawken, NJ); acrylates/steareth-20 methacrylate copolymer obtainable as Aculyn® 22 from International Specialty Products Corp. (Lombard, IL); and acrylates copolymer obtainable as either Aculyn® 33 from International Specialty Products Corp. or as Polymer EX-518® from BF Goodrich Corp. (Brecksville, OH); acrylates/steareth-20 itaconate copolymer, obtainable as Structure 2001®; acrylates/ceteth-20 itaconate copolymer, obtainable as Structure 3001®; and
acrylates/aminoacrylates/C10-30 alkyl PEG-20 itaconate copolymer, obtainable as Structure Plus® all from National Starch & Chemical, Inc. (Bridgewater, NJ).

The inventive compositions may be used for the cleansing of the user's skin and hair and is applied to a surface (e.g. a skin surface) via topical applications to release or deposit an effective amount of the transparent composition to perform the desired cleansing function. The frequency of topical application can vary widely, depending on the user's need. With respect to personal application to the skin, such application can range from about once per day to about four times per day, preferably from about twice a day to about three times a day.

The following examples are intended to illustrate the invention and not limit the invention in any way.

Several inventive transparent shower gels with suspended insoluble particles were prepared and compared to comparative shower gels that did not display clarity. The compositions of these shower gels are summarised in Tables 1 and 2. The processing methods used to prepare these compositions listed in Table 1 are provided below:

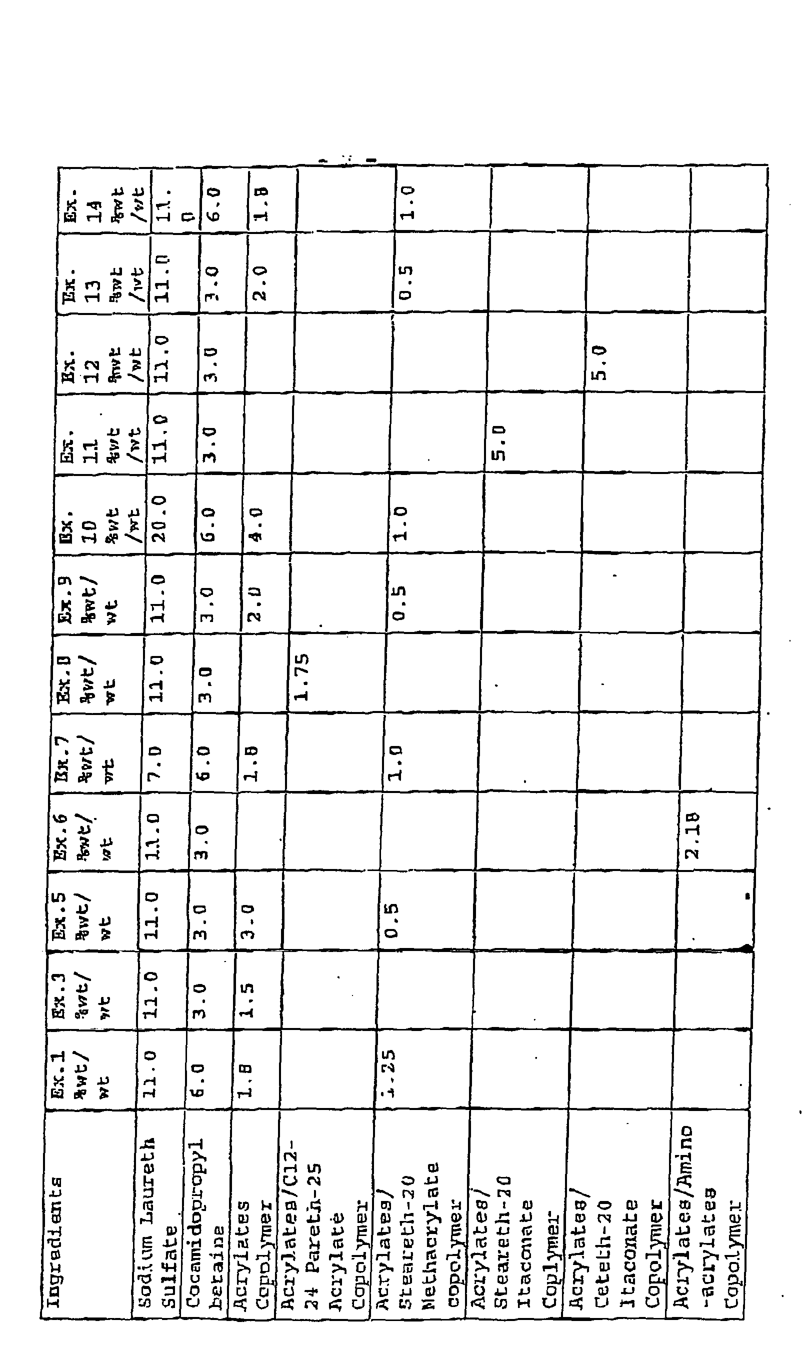

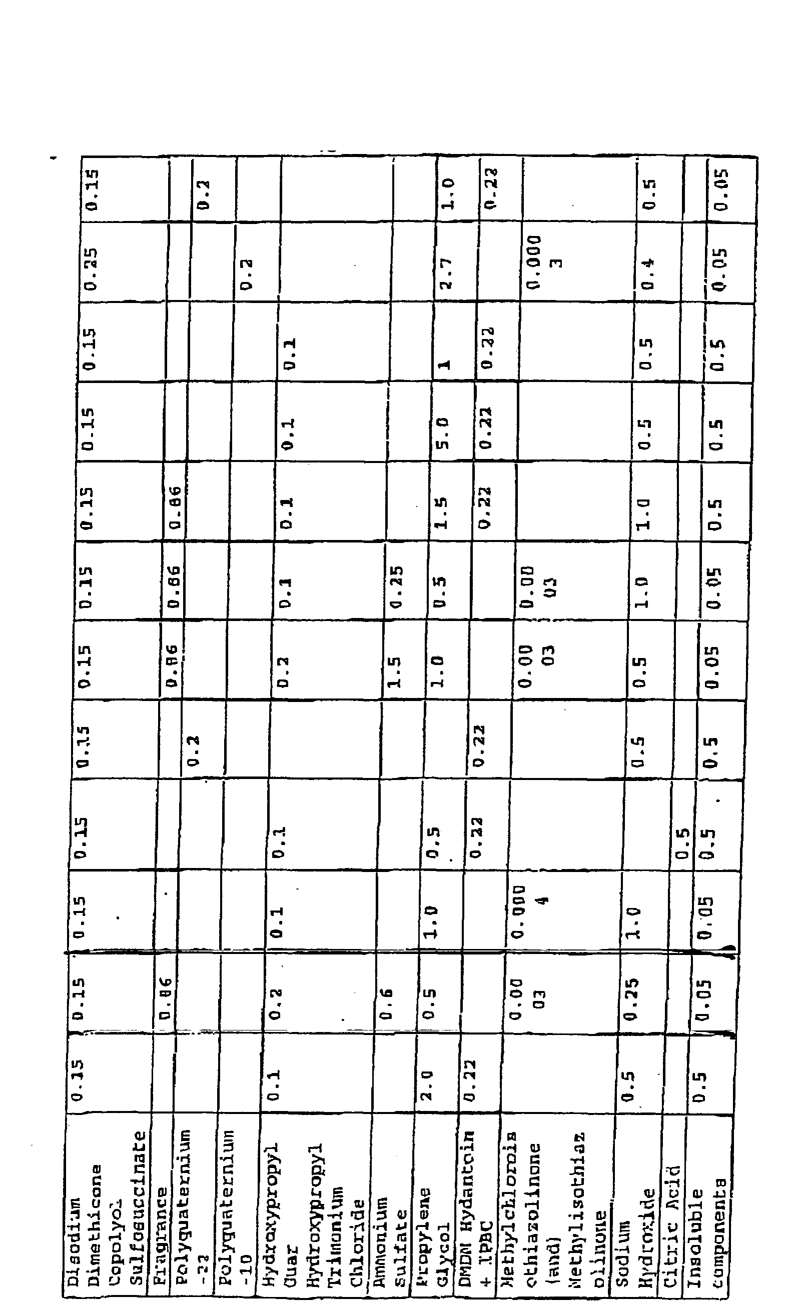

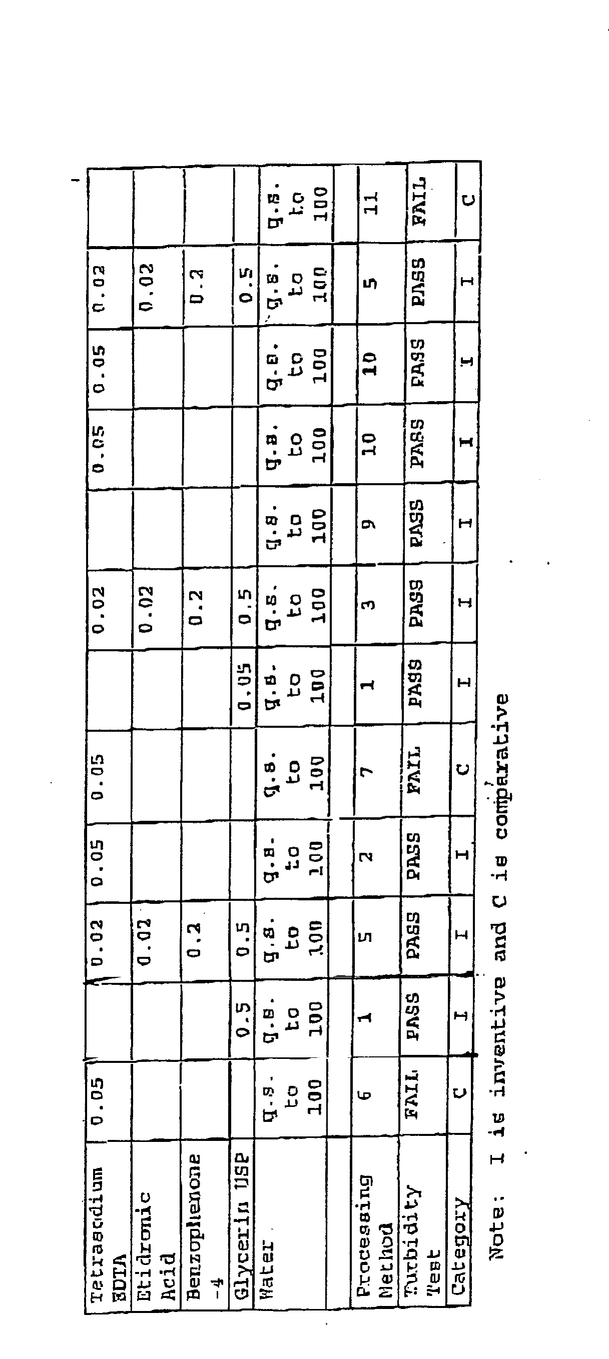

### METHOD 1:

The initial distilled water charge was added to a center turbine tank with wall scrape agitation and heated to 73.8°C (165°F). The temperature was maintained at 73.8°C (165°F). Agitation of the center turbine was increased as was the wall sweep so that there was a slight vortex. Acrylate copolymer was added to the tank and mixed. The anionic surfactants were then added to the tank and mixed, and then the amphoteric surfactant was added and mixed. The cationic polymer was premixed with propylene glycol if the cationic polymer was a solid, and was mixed well with no lumps. If the cationic polymer was a liquid, it was added straight to the tank. Then the glycerin was added. Agitation was decreased, and mixing was continued for 30 minutes at 73.8°C (165°F) and then cooled to 35°C (95 °F). During the cooling process, the pH was measured and adjusted with an alkaline pH adjuster to clarity within a target pH range of 5.5 to 7.0.

At 46.1°C (115 °F), the preservative was added and at 40.6°C (105 °F) the fragrance was added and mixed well. The mixture was then cooled to 35°C (95°F). At 35°C (95°F), the viscosity was measured and adjusted with ammonium sulfate to the desired viscosity. The insoluble components were added and mixed gently. The mixture was then cooled to room temperature.

### METHOD 2:

The initial distilled water charge was added to a center turbine tank with wall scrape agitation and heated to and maintained at 73.3°C (165°F). The agitation of the center turbine and the wall sweep was increased so that there was a slight vortex. Tetrasodium EDTA was added to the tank and mixed. The cationic polymer was premixed with propylene glycol if the cationic polymer was a solid, and mixed well with no lumps. If the cationic polymer was a liquid, it was added straight to the tank. The anionic surfactants were added and mixed. Next the amphoteric surfactants were added and mixed, and then the acrylate copolymer was added and mixed. The composition was then mixed for 30 minutes at 73.8°C (165°F), and was then cooled to 35°C (95 °F). At 46.1°C (115°F) , the preservative was added and mixed well. At 35°C (95 °F), the pH was measured and adjusted with citric acid to clarity within a target pH range of 5.5 to 7.0. At 35°C (95 °F), the viscosity was measured and adjusted if necessary to the desired viscosity. The insoluble components were then added and mixed, and the composition cooled to room temperature.

### METHOD 3:

The initial distilled water charge was added to a center turbine tank with wall scrape agitation and heated to and maintained at 73.8°C (165°F). Agitation of the center turbine and the wall sweep was increased so that there was a slight vortex. The acrylate copolymer was added to the tank and mixed. The anionic surfactant was added and mixed. Agitation was decreased and the amphoteric surfactant added and mixed. The cationic polymer was premixed with propylene glycol if the cationic polymer was a solid, and mixed well with no lumps. If the cationic polymer was a liquid, it was added straight to the tank. The remaining acrylate copolymer was premixed with water to a dilution of 4.5 to 1 and added to the tank and mixed with gentle agitation. The batch was mixed for 30 minutes at 73.8°C (165°F) and was started to be cooled to 35°C (95 °F). At 48.9°C (120 °F), the glycerin was added, at 46.1°C (115 °F), the preservative and then the UV inhibitor were added. At 43.3°C (110 °F), the EDTA and the EHDP were added and at 40.6°C (105 °F), the fragrance was added and mixed. At 35°C (95 °F), the pH was measured and adjusted with an alkaline pH adjuster to clarity within a target pH range of 5.9 to 7.0. At 35°C (95 °F), the viscosity was measured and adjusted with propylene glycol to the desired viscosity. The insoluble components were then added and mixed gently, and the composition cooled to room temperature.

### METHOD 4:

The initial distilled water charge was added to a center turbine tank with wall scrape agitation and heated to and maintained at 46.1°C (115°F). Agitation of the center turbine and the wall sweep was increased so that there was a slight vortex. Preservative was added to the tank and mixed. The acrylate copolymer was added to the tank slowly and mixed for 5 minutes. The anionic surfactants were added, then the amphoteric surfactants and mixed. The batch was cooled to 35°C (95 °F) . At 35°C (95 °F), the pH was measured and adjusted with an alkaline pH adjuster to a target pH range of 5.5 to 7.0. At 35°C (95 °F), the viscosity was measured and adjusted with propylene glycol to the desired viscosity. The insoluble components were added and mixed, and the composition cooled to room temperature.

### METHOD 5:

The initial distilled water charge was added to a center turbine tank with wall scrape agitation and heated to and maintained at 73.8°C (165°F). Agitation of the center turbine and the wall sweep was increased so that there was a slight vortex. The acrylate copolymer was added to the tank and mixed. The anionic surfactant was then added and mixed. Agitation was decreased, and the amphoteric surfactant added and mixed. The cationic polymer was premixed with propylene glycol if the cationic polymer was a solid, and mixed well with no lumps. If the cationic polymer was a liquid, it was added straight to the tank. The remaining acrylate copolymer was premixed with water to a dilution of 4.5 to 1, added to the tank and mixed with gentle agitation. The batch was mixed for 30 minutes at 73.8°C (165°F), and then started to cool to 35°C (95 °F) . At 48.9°C (120 °F) , the glycerin was added, at 46.1°C (115 °F) the preservative and then the UV inhibitor were added. At 43.3°C (110 °F), the EDTA and the EHDP were added and mixed. At 35°C (95 °F), the pH was measured and adjusted with an alkaline pH adjuster to clarity within a target pH range of 5.9 to 7.0. At 35°C (95 °F), the viscosity was measured and adjusted with propylene glycol to the desired viscosity. The insoluble components were then added and mixed gently, and the composition cooled to room temperature.

### METHOD 6:

The initial distilled water charge was added to a center turbine tank with wall scrape agitation and heated to and maintained at 73.8°C (165°F). Agitation of the center turbine and the wall sweep was increased so that there was a slight vortex. Tetrasodium EDTA was added to the tank. The cationic polymer was premixed with propylene glycol if the cationic polymer was a solid, and mixed well with no lumps. If the cationic polymer was a liquid, it was added straight to the tank. Agitation was decreased, and the acrylate copolymer(s) added and mixed. The anionic surfactants were added to the tank and mixed. The amphoteric surfactant was added next and mixed. This was mixed for 30 minutes at 73.8°C (165°F) and then cooled to 35°C (95 °F). At 46.1°C (115°F), the preservative was added and mixed well. At 35°C (95 °F), the pH was measured and adjusted with an alkaline pH adjuster to clarity within a target pH range of 5.5 to 7.0. At 35°C (95 °F), the viscosity was measured and adjusted with propylene glycol to the desired viscosity. The insoluble components were added and mixed, and the composition cooled to room temperature.

### METHOD 7:

The initial distilled water charge was added to a center turbine tank with wall scrape agitation and heated to and maintained at 73.8°C (165°F). Agitation of the center turbine and the wall sweep was increased so that there was a slight vortex. Tetrasodium EDTA was added to the tank and then subsequently the acrylate copolymer(s). The anionic surfactants were then added to the tank and mixed. The amphoteric surfactant was added next and mixed. The cationic polymer was premixed with propylene glycol if the cationic polymer was a solid, and mixed well with no lumps. If the cationic polymer was a liquid, it was added straight to the tank. Agitation was decreased and the composition was mixed for 30 minutes at 73.8°C (165°F) and then was cooled to 35°C (95 °F). At 46.1°C (115 °F), the preservative were added and mixed well. At 35°C (95 °F), the pH was measured and adjusted with an alkaline pH adjuster to clarity within a target pH range of 5.5 to 7.0. At 35°C (95 °F), the viscosity was measured and adjusted with propylene glycol to the desired viscosity. The insoluble components were added and mixed, and the composition cooled to room temperature.

### METHOD 8:

The initial distilled water charge was added to a center turbine tank with wall scrape agitation and heated to and maintained at 73.8°C (165°F). Agitation of the center turbine and the wall sweep was increased so that there was a slight vortex. The acrylate copolymer was added to the tank and mixed. The anionic surfactant was added and mixed. Agitation was decreased and the amphoteric surfactant added and mixed. The cationic polymer was premixed with propylene glycol if the cationic polymer was a solid, and mixed well with no lumps. If the cationic polymer was a liquid, it was added straight to the tank. The remaining acrylate copolymer was premixed with water to a dilution of 4.5 to 1 and added to the tank and mixed with gentle agitation. The batch was mixed for 30 minutes at 73.8°C (165°F) and cooled to 35°C (95 °F) . At 48.9°C (120°F) the glycerin was added, and at 46.1°C (115 °F) the preservative and then the UV inhibitor were added. At 43.3°C (110 °F), the EDTA and the EHDP were added and mix. At 40.5°C (105 °F) the fragrance was added and mixed well. At 35°C (95 °F), the pH was measured and adjusted with an alkaline pH adjuster to clarity within a target pH range of 5.9 to 7.0. At 35°C (95 °F), the viscosity was measured and adjusted with propylene glycol to the desired viscosity. The insoluble components were added and mix, and the composition cooled to room temperature.

### METHOD 9:

The initial distilled water charge was added to a center turbine tank with wall scrape agitation and heated to and maintained at 73.8°C (165°F). Agitation of the center turbine and the wall sweep was increased so that there is a slight vortex. The acrylate copolymer was added to the tank and mixed. The anionic surfactant was added and mixed. Agitation was decreased, and the amphoteric surfactant added and mix. The cationic polymer was premixed with propylene glycol if the cationic polymer was a solid, and mixed well with no lumps. If the cationic polymer was a liquid, it was added straight to the tank. The remaining acrylate copolymer was premixed with water to a dilution of 4.5 to 1 and added to the tank and mixed with gentle agitation. The batch was mixed for 30 minutes at 73.8°C (165°F) and was then cooled to 35°C (95 °F). At 46.1°C (115 °F) the preservative was added, and at 40.6°C (105 °F) the fragrance was added and mixed. At 35°C (95 °F), the pH was measured and adjusted with an alkaline pH adjuster to clarity within a target pH range of 5.9 to 7.0. At 35°C (95 °F) , the viscosity was measured and adjusted with propylene glycol to the desired viscosity. The insoluble components were added and mixed gently, and the composition cooled to room temperature.

### METHOD 10 :

The initial distilled water charge was added to a center turbine tank with wall scrape agitation and heated to and maintained at 73.8°C (165°F). Agitation of the center turbine and the wall sweep was increased so that there was a slight vortex. EDTA was added to the tank and mixed. The cationic polymer was premixed with propylene glycol if the cationic polymer was a solid, and mixed well with no lumps. If the cationic polymer was a liquid, it was added straight to the tank. The anionic surfactant was added and mixed. The amphoteric surfactant was added and mixed. The acrylate copolymer was added to the tank and mixed. The agitation was decreased and the batch mixed for 30 minutes at 73.8°C (165°F) and then cooled to 35°C (95 °F) . At 46. 1°C (115 °F), the preservative was added and mixed. At 35°C (95 °F), the pH was measured and adjusted with an alkaline pH adjuster to clarity within a target pH range of 5.9 to 7.0. At 35°C (95 °F), the viscosity was measured and adjusted with propylene glycol to the desired viscosity. The insoluble components were added and mixed, and the composition cooled to room temperature.

### METHOD 11:

The initial distilled water charge was added to a center turbine tank with wall scrape agitation and heated to and maintained at 73.8°C (165°F). Agitation of the center turbine and the wall sweep was increased so that there was a slight vortex. EDTA and then the acrylate copolymers were added to the tank and mixed. The anionic surfactant was added and mixed. The amphoteric surfactant was added and mixed. The cationic polymer was premixed with propylene glycol if the cationic polymer was a solid, and mixed well with no lumps. If the cationic polymer was a liquid, it was added straight to the tank. The agitation was decreased and the batch mixed for 30 minutes at 73. 8°C (165°F) , and then cooled to 35°C (95 °F) . At 46. 1°C (115°F), the preservative was and mixed. At 35°C (95 °F), the pH was measured and adjusted with an alkaline pH adjuster to clarity within a target pH range of 5.9 to 7.0. At 35°C (95 °F), the viscosity was measured and adjusted with propylene glycol to the desired viscosity. The insoluble components were added and mixed, and the composition cooled to room temperature.

### Example 15

The following is another example of the invention.

| **Ingredient** | **% by weight** |
|---|---|
| Anionic surfactant (e.g. Sodium Laureth Sulfate (3EO) | About 10-20 |
| Betaine (e.g. Cocoamidopropyl betaine) | About 2-15 |
| Acrylate Copolymer (e.g. Aculyn 33 and 22) | About 2-15 |
| Silicone (e.g. Dimethicone Copolyol Sulfosuccinate | About 0.1 - 5 |
| Fragrance | About 0 - 1.0 |
| Cationic surfactant (e.g. Hydroxypropyl Guar Hydroxypropyl Trimonium Chloride) | About .05 - 5 |
| Propylene Glycol | About 0.1 - 2.0 |
| Preservative | About 0.1 - 2.0 |
| Sodium Hydroxide | to adjust pH to 6.0 to 7.0 |
| Beads (e.g. Agar/ Titanium Dioxide/Sunflower Oil Beads | About 0.1 - 2.0 |
| Water | q.s. to 100 |

### METHODS

### Viscosity

For the purposes of this invention, viscosity is measured using conventional techniques with a Brookfield viscometer, Model HBDVII+ CP, spindle No. 41 at 0.5 rpm at 25°C.

### Turbidity

For the purposes of this invention, the acceptability of formulation clarity was measured qualitatively and quantitively using a visual method of turbidity determination and a turbidimeter respectively. Briefly, the visual method involves looking through a determined path length of the formulation to a visual target and determining if the visual target is legible or recognizable. This target may be a straight line, a set of parallel lines, or a number or letter printed on white paper. To assess turbidity, the test formulation was placed in a glass beaker such that the height from the bottom of the beaker to the top surface of the formulation was 10.16 cm (four inches). The formulation is made free of air bubbles. A piece of paper with the visual target is placed under the beaker. The assessor the looked through the top surface of the formulation to the visual target. If the visual target appeared similar to the original, the formulation is of acceptable clarity and receives a 'pass' rating. If the visual target appeared significantly hazy, or is out of focus compared to the original target, the formulation is of unacceptable clarity and receives a 'fail' rating.

Turbidity was quantitatively determined by a Turbidimeter, Model DRT-100D, manufactured by Shaban Manufacturing Inc, H. F. Instruments Division using a sample cuvette of 28 mm diameter by 91 mm in length with a flat bottom. Samples that had received a 'pass' rating from the visual method were found to have a turbidity measurement of less than or equal to 105 NTU's (Nephelometric Turbidity Units). Samples that had received a 'fail' rating from the visual method were found to have a turbidity measurement of greater than 105 NTU' s .

The foregoing description and examples illustrate selected embodiments of the present invention. In light thereof, variations and modifications will be suggested to one skilled in the art, all of which are written the scope and spirit of this invention.

## Claims

1. A transparent cleansing composition, comprising:
a. 5 to 30 weight percent of at least one anionic surfactant:
b. 2 to 15 weight percent of at least one amphoteric surfactant;
c. 0.1 to 10 weight percent of at least one at least partially neutralised acrylate copolymer;
d. 0.01 to 5 weight percent of at least one cationic polymer;
e. 0.01 to 5 weight percent of at least one insoluble component selected from beads, particulates, water insoluble liquids and gas bubbles, and mixtures thhereof;
f. 50 to 85 weight percent of water;
g. wherein there is a weight percentage ratio range of anionic surfactant to amphoteric surfactant of 1.9:1 to 15:1;
h. wherein there is a weight percent ratio range of the sum of cationic polymer and amphoteric surfactant to acrylate copolymer of 0.1:1 to 15:1; and
wherein the concentration of acrylate copolymer is sufficient to suspend said at least one insoluble component.

2. The cleansing composition of claim 1, comprising:
a. B to 20 weight percent of at least one anionic surfactant;
b. Z to 10 weight percent of at least one amphoteric surfactant;
c. 0.5 to 5 weight percent of at least one at least partially neutralised acrylate copolymer;
d. 0.1 to 2 weight percent of at least one cationic polymer;
e. 0.05 to 3 weight percent of at least one insoluble component selected from the group consisting of beads, particulates, water insoluble liquids and gas bubbles ;
f. 50 to 85 weight percent of water;
g. wherein there is a weight percent ratio range of anionic surfactant to amphoteric surfactant of 1.9:1 to 10:1;
h. wherein there is a weight percent ratio range of the sum of cationic polymer and amphoteric surfactant to acrylate copolyer of 0-3:1 to 10:1; and
wherein the viscosity range is between 600D and 200D0 cps, and the pH is in the range of 5.5 to 7.0.

3. The cleansing composition of claim 1 or claim 2 wherein the anionic surfactant is selected from alkyl sulfates, alkyl ether sulfates, alkyl sulfonates, alkylbenzene sulfonates, alkyl succinates, alkyl sulfosuccinates, alkyl olefin sulfonates, alkyl sarcosinates, octoxynol phosphates nonoxynol phosphates, alkyl taurates, polyoxyethylene sulfates, polyoxyethylene isethionates, alkyl carboxylates and alkyl ether carboxylates, and mixtures thereof.

4. The cleansing composition of any of the preceding claims wherein the amphoteric surfactant is selected from alkyl betaines, alkyl amino betaines, hydroxysultaines, alkyl amphoacetates and alkylampho carboxyglycinates, and mixtures thereof.

5. The cleansing composition of any of the preceding claims wherein the at least one acrylate copolymer includes one or more copolymers containing at least one monomer selected from methacrylic acid, acrylic acid, amino acrylic acid, an acrylic acid ester of a C8 -30 alkyl, alkylaryl, aryl, heterocyclic, alkoxyl, alkoxyl alkyl ester of a C8-30 alkyl or alkenyl; either substituted or unsubstituted; a methacrylic acid ester of a C8 -C30 alkyl, alkylaryl, aryl, heterocyclic, alkoxyl, alkoxyl alkyl ester of a C8-30 alkyl, or alkenyl; either substituted or unsubstituted; a C1-4 alkyl acrylate, and a C1-4 methacrylate; either substituted or unsubstituted.

6. The cleansing composition of any of the preceding claims wherein the cationic polymer is selected from quaternized guar gums, quaternized phosphate esters, quaternized polysaccharides or their derivatives, quaternized polyamides, quaternized polymeric derivatives of acrylates, methacrylates, acrylamides, methacrylamides or copolymers thereof, and quaternized polymeric derivatives of substituted allyl or vinyl compounds.

7. The cleansing composition of any of the preceding claims wherein the at least one insoluble component is selected from glass beads, plastic beads, macaroni food products, organic materials, inorganic materials, crystalline solids, oil droplets, vegetable and fruit purees, water insoluble dimethicones, air and gas bubbles, and mixtures thereof.

8. A method of cleaning the skin or hair with a transparent cleansing product according to any preceding claim.

## Patentansprüche

1. Transparente Reinigungszusammensetzung, umfassend:
a. 5 bis 30 Gew.-% mindestens eines anionischen Tensids;
b. 2 bis 15 Gew.-% mindestens eines amphoteren Tensids;
c. 0,1 bis 10 Gew.-% mindestens eines mindestens teilweise neutralisierten Acrylat-Copolymers;
d. 0,01 bis 5 Gew.-% mindestens eines kationischen Polymers;
e. 0,01 bis 5 Gew.-% mindestens einer unlöslichen Komponente, ausgewählt aus Kugeln, teilchenförmigen Stoffen, in Wasser unlöslichen Flüssigkeiten und Gasblasen und Gemischen davon;
f. 50 bis 85 Gew.-% Wasser;
g. worin es einen Verhältnisbereich in Gewichtsprozent von anionischem Tensid zu amphoterem Tensid von 1,9:1 bis 15:1 gibt;
h. worin es einen Verhältnisbereich in Gewichtsprozent von der Summe von kationischem Polymer und amphoterem Tensid zu Acrylat-Copolymer von 0,1:1 bis 15:1 gibt; und
worin die Konzentration von Acrylat-Copolymer ausreichend ist, um die mindestens eine unlösliche Komponente zu suspendieren.

2. Reinigungszusammensetzung nach Anspruch 1, umfassend:
a. 8 bis 20 Gew.-% mindestens eines anionischen Tensids;
b. 2 bis 10 Gew.-% mindestens eines amphoteren Tensids;
c. 0,5 bis 5 Gew.-% mindestens eines mindestens teilweise neutralisierten Acrylat-Copolymers;
d. 0,1 bis 2 Gew.-% mindestens eines kationischen Polymers;
e. 0,05 bis 3 Gew.-% mindestens einer unlöslichen Komponente, ausgewählt aus der Gruppe, bestehend aus Kugeln, teilchenförmigen Stoffen, in Wasser unlöslichen Flüssigkeiten und Gasblasen;
f. 50 bis 85 Gew.-% Wasser;
g. worin es einen Verhältnisbereich in Gewichtsprozent von anionischem Tensid zu amphoterem Tensid von 1,9:1 bis 10:1 gibt;
h. worin es einen Verhältnisbereich in Gewichtsprozent von der Summe von kationischem Polymer und amphoterem Tensid zu Acrylat-Copolymer von 0,3:1 bis 10:1 gibt; und
worin der Viskositätsbereich zwischen 6000 und 20000 cP liegt und der pH-Wert im Bereich von 5,5 bis 7,0 liegt.

3. Reinigungszusammensetzung nach Anspruch 1 oder Anspruch 2, worin das anionische Tensid aus Alkylsulfaten, Alkylethersulfaten, Alkylsulfonaten, Alkylbenzolsulfonaten, Alkylsuccinaten, Alkylsulfosuccinaten, Alkylolefinsulfonaten, Alkylsarcosinaten, Octoxynolphosphaten, Nonoxynolphosphaten, Alkyltauraten, Polyoxyethylensulfaten, Polyoxyethylenisethionaten, Alkylcarboxylaten und Alkylethercarboxylaten und Gemischen davon ausgewählt ist.

4. Reinigungszusammensetzung nach einem der vorangehenden Ansprüche, worin das amphotere Tensid aus Alkylbetainen, Alkylaminobetainen, Hydroxysultainen, Alkylamphoacetaten und Alkylamphocarboxyglycinaten und Gemischen davon ausgewählt ist.

5. Reinigungszusammensetzung nach einem der vorangehenden Ansprüche, worin das mindestens eine Acrylat-Copolymer ein oder mehrere Copolymere, enthaltend mindestens ein Monomer, ausgewählt aus Methacrylsäure, Acrylsäure, Aminoacrylsäure, einem Acrylsäureester von einem C8-30-Alkyl, Alkylaryl, Aryl, Heterocyclus, Alkoxyl, Alkoxylalkylester von einem C8-30-Alkyl oder Alkenyl; entweder substituiert oder unsubstituiert; einem Methacrylsäureester von einem C8-30-Alkyl, Alkylaryl, Aryl, Heterocyclus, Alkoxyl, Alkoxylalkylester von einem C8-30-Alkyl oder Alkenyl; entweder substituiert oder unsubstituiert; einem C1-4-Alkylacrylat und einem C1-4-Methacrylat; entweder substituiert oder unsubstituiert, einschließt.

6. Reinigungszusammensetzung nach einem der vorangehenden Ansprüche, worin das kationnische Polymer aus quaternisierten Guargummen, quaternisierten Phosphatestern, quaternisierten Polysacchariden oder deren Derivaten, quaterinisierten Polyamiden, quaternisierten polymeren Derivaten von Acrylaten, Methacrylaten, Acrylamiden, Methacrylamiden oder Copolymeren davon und quaternisierten polymeren Derivaten von substituierten Allyl- oder Vinylverbindungen ausgewählt ist.

7. Reinigungszusammensetzung nach einem der vorangehenden Ansprüche, worin die mindestens eine unlösliche Komponente aus Glaskugeln, Kunststoffkugeln, Makkaroninahrungsprodukten, organischen Materialien, anorganischen Materialien, kristallinen Feststoffen, Öltröpfchen, Gemüse- und Obstpürees, in Wasser unlöslichen Dimethiconen, Luft- und Gasblasen und Gemischen davon ausgewählt ist.

8. Verfahren zum Reinigen von Haut oder Haar mit einem transparenten Reinigungsprodukt nach einem vorangehenden Anspruch.

## Revendications

1. Composition lavante transparente comprenant :
a. 5 à 30 % en poids d'au moins un tensioactif anionique ;
b. 2 à 15 % en poids d'au moins un tensioactif amphotère ;
c. 0,1 à 10 % en poids d'au moins un copolymère d'acrylate au moins partiellement neutralisé ;
d. 0,01 à 5 % en poids d'au moins un polymère cationique ;
e. 0,01 à 5 % en poids d'au moins un composant non soluble sélectionné parmi les billes, les particules, les liquides non hydrosolubles et les bulles de gaz, et les mélanges de ceux-ci ;
f. 50 à 85 % en poids d'eau ;
g. dans lequel le ratio de pourcentage en poids entre le tensioactif anionique et le tensioactif amphotère se situe dans la plage de 1,9:1 à 15:1 ;
h. dans lequel le ratio de pourcentage en poids entre la somme du polymère cationique et du tensioactif amphotère et le copolymère d'acrylate se situe dans la plage de 0,1:1 à 15:1 ; et
dans lequel la concentration en copolymère d'acrylate est suffisante pour mettre en suspension ledit au moins un composant non soluble.

2. Composition lavante selon la revendication 1, comprenant :
a. 8 à 20 % en poids d'au moins un tensioactif anionique ;
b. 2 à 10 % en poids d'au moins un tensioactif amphotère ;
c. 0,5 à 5 % en poids d'au moins un copolymère d'acrylate au moins partiellement neutralisé ;
d. 0,1 à 2 % en poids d'au moins un polymère cationique ;
e. 0,05 à 3 % en poids d'au moins un composant non soluble sélectionné dans le groupe constitué des billes, particules, liquides non hydrosolubles et bulles de gaz ;
f. 50 à 85 % en poids d'eau ;
g. dans lequel le ratio de pourcentage en poids entre le tensioactif anionique et le tensioactif amphotère se situe dans la plage de 1,9:1 à 10:1 ;
h. dans lequel le ratio de pourcentage en poids entre la somme du polymère cationique et du tensioactif amphotère et le copolymère d'acrylate se situe dans la plage de 0,3:1 à 10:1 ; et
dans lequel la plage de viscosité est entre 6000 et 20000 cps, et le pH est dans la plage de 5,5 à 7,0.

3. Composition lavante selon la revendication 1 ou la revendication 2, dans laquelle le tensioactif anionique est choisi parmi les sulfates d'alkyle, éthersulfates d'alkyle, sulfonates d'alkyle, sulfonates d'alkylbenzène, succinates d'alkyle, sulfosuccinates d'alkyle, oléfinesulfonates d'alkyle, sarcosinates d'alkyle, phosphates d'octoxynol, phosphates de nonoxynol, taurates d'alkyle, sulfates de polyoxyéthylène, iséthionates de polyoxyéthylène, carboxylates d'alkyle et éther carboxylates d'alkyle, et les mélanges de ceux-ci.

4. Composition lavante selon l'une quelconque des revendications précédentes, dans laquelle le tensioactif amphotère est sélectionné parmi les bétaïnes d'alkyle, aminobétaïnes d'alkyle, hydroxysultaïnes, amphoacétates d'alkyle, amphocarboxyglycinates d'alkyle, et les mélanges de ceux-ci.

5. Composition lavante selon l'une quelconque des revendications précédentes, dans laquelle le au moins un copolymère d'acrylate inclut un ou plusieurs copolymères contenant au moins un monomère sélectionné parmi l'acide méthacrylique, l'acide acrylique, l'acide amino acrylique, un ester d'acide acrylique d'un alkyle en C₈ - C₃₀, d'un alkylaryle, d'un aryle, d'un hetérocyclique, d'un alcoxyle, d'un ester alkoxylalkylique d'un alkyle ou alcényle en C₈ - C₃₀ ; soit substitués soit non substitués ; un ester d'acide méthacrylique d'un alkyle en C₈ - C₃₀, d'un alkylaryle, d'un aryle, d'un hetérocyclique, d'un alkoxyle, d'un ester alkoxylalkylique d'un alkyle ou d'un alcényle en C₈ - C₃₀ ; un acrylate alkylique en C₁ - C₄ et un méthacrylate en C₁ - C₄ ; soit substitué soit non substitué.

6. Composition lavante selon l'une quelconque des revendications précédentes dans laquelle le polymère cationique est sélectionné parmi les gommes de guar quaternisées, les esters de phosphate quaternisés, les polysaccharides quaternisés ou leurs dérivés, les polyamides quaternisés, les dérivés polymères quaternisés d'acrylates, de méthacrylates, d'acrylamides, de méthacrylamides ou de copolymères de ceux-ci, et les dérivés polymères quaternisés de composés allyle ou vinyle substitués.

7. Composition lavante selon l'une quelconque des revendications précédentes dans laquelle le au moins un composant non soluble est sélectionné parmi les billes de verre, les billes de plastiques, les macaronis alimentaires, les matériaux organiques, les matériaux inorganiques, les solides cristallins, les gouttelettes d'huile, les purées de légumes et de fruits, les diméthicones non hydrosolubles, les bulles d'air et de gaz, et les mélanges de ceux-ci.

8. Méthode pour laver la peau ou les cheveux avec un produit lavant transparent selon l'une quelconque des revendications précédentes.
